(19) Europäisches Patentamt
European Patent Office
Office européen des brevets

(11) Numéro de publication : **0 094 283**
**B1**

(12) **FASCICULE DE BREVET EUROPÉEN**

(45) Date de publication du fascicule du brevet : **21.11.85**

(21) Numéro de dépôt : **83400829.4**

(22) Date de dépôt : **27.04.83**

(51) Int. Cl.[4] : **A 61 K 31/52**, C 07 D263/04, C 07 C103/78 // (A61K31/52, 31:415),(A61K31/52, 31:165)

(54) **Forme médicamenteuse de théophylline à action prolongée.**

(30) Priorité : **04.05.82 FR 8207695**
**14.04.83 FR 8306075**

(43) Date de publication de la demande :
**16.11.83 Bulletin 83/46**

(45) Mention de la délivrance du brevet :
**21.11.85 Bulletin 85/47**

(84) Etats contractants désignés :
**AT BE CH DE FR GB IT LI LU NL SE**

(56) Documents cités :
**FR-A- 2 040 181**
**CHIMIE THERAPEUTIQUE, vol. VIII, no. 2, mars-avril 1973, pages 202-206, Paris, FR. M. BAYSSAT et al.: "Nouvelles hydroxyalkyl cinnamamides à activité myorelaxante"**
**CHEMICAL ABSTRACTS, vol. 92, no. 25, 23 juin 1980, page 13, no. 208747f, Columbus, Ohio, USA J.L. BRAZIER et al.: "Inhibition by idrocilamide of the disposition of caffeine"**
**EUROPEAN JOURNAL OF MEDICINAL CHEMISTRY, CHIMICA THERAPEUTICA, vol. IX, no. 5, septembre-octobre 1974, pages 543-547, Chatenay-Malabry, FR. M. BELLEVILLE et al.: "Taux sanguins et élimination urinaire d'un nouveau myorelaxant le N-(hydroxy-2 éthyl) cinnamamide (LCB.29)"**

(73) Titulaire : **LIPHA, LYONNAISE INDUSTRIELLE PHARMACEUTIQUE**
**34, rue Saint Romain Boîte Postale 8481**
**F-69359 Lyon Cedex 08 (FR)**

(72) Inventeur : **Bayssat, Michel**
**Chemin de Beckensteiner**
**F-69260 Charbonnieres (FR)**
Inventeur : **Belleville, Maryse**
**27, avenue Lacassagne**
**F-69003 Lyon (FR)**
Inventeur : **Grand, Marcel**
**15, rue du Professeur Nicolas**
**F-69008 Lyon (FR)**

(74) Mandataire : **Bouton Neuvy, Liliane et al**
**L'Air liquide, Société Anonyme pour L'Etude et L'Exploitation des Procédés Georges Claude 75, Quai d'Orsay**
**F-75321 Paris Cedex 07 (FR)**

**EP 0 094 283 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention représente une forme médicamenteuse de théophylline, euphylline® ou aminophylline® à action prolongée.

La théophylline, diméthyl-1,3 xanthine, se présente sous forme d'une poudre blanche de saveur amère, peu soluble dans l'eau, de formule

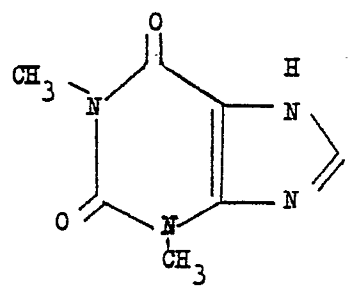

Elle possède un effet bronchodilatateur dû à une relaxation des muscles lisses bronchiques ; elle est active vis-à-vis de tous les agents bronchoconstricteurs. Elle exerce également une action inhibitrice vis-à-vis des mastocytes en inhibant partiellement leur dégranulation avec libération d'histamine dans les réactions d'hypersensibilité.

Comme, R. W. BUTCHER et E. W SUTHERLAND, J. Biol. Chem. tome 237, pages 1244 à 1250 (1962) l'ont montré, cette activité semble liée à une inhibition compétitive, sur la phosphodiestérase, enzyme qui catalyse la transformation de l'AMP cyclique (3'-5' Adénosine monophosphate cyclique) en 5'-AMP. Il en résulte une augmentation tissulaire de l'AMP cyclique qui serait responsable de l'activité de la théophylline.

La théophylline est utilisée depuis longtemps dans le traitement de l'asthme, mais son utilisation avait connu un certain discrédit, le produit étant considéré comme mal toléré et peu efficace. De nouvelles techniques de dosage, par chromatographie liquide haute performance et par les méthodes immuno-enzymologiques, permettent maintenant une détermination simple et précise des taux sanguins de théophylline. Ces méthodes ont fait progresser l'utilisation du médicament, et la théophylline reprend une place importante dans le traitement de l'asthme. Quoique l'utilisation des adrénocorticostéroïdes puisse parfois être nécessaire dans le traitement de la maladie, les médicaments bronchodilatateurs occupent une place dominante dans la thérapeutique et parmi eux la théophylline est sans doute le médicament le plus utile.

La théophylline est très bien absorbée par voie orale. Par contre, par voie rectale l'absorption est très irrégulière. L'administration intrapulmonaire par aérosol est inefficace.

On a établi une relation effet-concentration sanguine. Pour un taux sanguin compris entre 5 et 20 mg/l, l'amélioration de la capacité vitale pulmonaire est proportionnelle au logarithme de la concentration. Au-dessus d'une concentration de 20 mg/l peuvent apparaître des signes d'intolérance : désordres digestifs, troubles nerveux, troubles cardiaques puis atteinte neurologique.

La majeure partie de la théophylline est métabolisée au niveau hépatique pour donner des métabolites inactifs éliminés par le rein. Sa demi-vie est variable avec l'âge et avec les individus ; elle est en moyenne voisine de 6 heures chez l'adulte normal, mais des demi-vies de 4 heures ne sont pas exceptionnelles.

Pour assurer un bon résultat, il faudrait que les malades absorbent une prise de médicament toutes les 6 heures. Cependant, comme la maladie asthmatique revêt un caractère chronique, il n'est guère possible d'obtenir un traitement bien suivi avec des prises aussi fréquentes et régulièrement espacées dans la journée.

On a proposé diverses formulations galéniques — du type micro granules par exemple — à partir desquelles la théophylline se trouve lentement libérée dans l'appareil digestif.

Toutefois, il apparaît souhaitable d'obtenir des taux sanguins plus durables, ce qui permettrait de simplifier la posologie, en diminuant le nombre de prises médicamenteuses, et aussi en diminuant les fluctuations des taux sanguins.

Il a été recherché une association de la théophylline, euphylline® ou aminophylline® avec un produit maintenant son taux sanguin dans des limites thérapeutiques favorables avec une action prolongée.

Il a été trouvé une association de théophylline, euphylline® ou aminophylline® qui pallie l'inconvénient de la demi-vie relativement courte de la théophylline, et permet d'obtenir après une prise un taux sanguin de théophylline stable et prolongé, avec une efficacité thérapeutique de 24 heures sur 24.

Selon l'invention la théophylline, euphylline® ou aminophylline® est associée à un composé du type cinnamamide substitué répondant à la formule générale

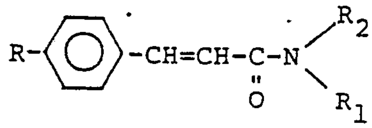

(I)

dans laquelle R est l'hydrogène, un halogène ou le radical acétyle ; $R_1$ est l'hydrogène ou le groupe méthyle ; $R_2$ représente un groupe de formule II :

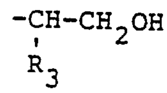

dans laquelle $R_3$ est l'hydrogène ou le radical méthyle ; $R_1$ et $R_2$ peuvent également former avec l'atome d'azote adjacent le groupe diméthyl-2,2 oxazolidinyl-3 de formule :

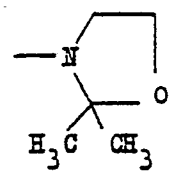

(III)

Parmi les composés de la famille des cinnamamides substitués associables à la théophylline on peut citer notamment les dérivés N-(hydroxy-2 éthyl) cinnamamide, N-(hydroxy-2 éthyl) chloro-4 cinnamamide, N-méthyl, N-(hydroxy-2 éthyl) cinnamamide, N-[(hydroxy-1 propyl)-2] cinnamamide, N-(hydroxy-2 éthyl) fluoro-4 cinnamamide, N-(hydroxy-2 éthyl) acétyl-4 cinnamamide, (chloro-4 cinnamoyl)-3 diméthyl-2,2 oxazolidine, diméthyl-2,2 (fluoro-4 cinnamoyl)-3 oxazolidine et cinnamoyl-3 diméthyl-2,2 oxazolidine.

Les dérivés pour lesquels $R_2$ est le groupe représenté par la formule II s'obtiennent selon les méthodes décrites dans le brevet français 2.040.181, au nom de Lipha, Lyonnaise Industrielle Pharmaceutique. On peut, par exemple, avantageusement condenser un chlorure de cinnamoyle de formule IV

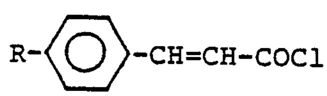

(IV)

avec une aminé de formule générale

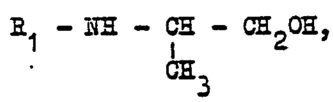

(V)

dans laquelle R, $R_1$ et $R_3$ ont les mêmes significations que précédemment. La réaction s'effectue dans un solvant inerte tel le dioxanne, le tétrahydrofuranne ou le chloroforme, en présence d'un agent alcalin qui peut être un carbonate ou bicarbonate alcalin, une base organique comme la pyridine ou la triéthylamine ou encore un excès de l'amine de formule V. La réaction s'effectue à des températures comprises entre 0 °C et la température d'ébullition du solvant utilisé.

Le composé I : N-(hydroxy-2 éthyl) cinnamamide $C_{11}H_{13}NO_2$, M = 191,22, cristallisé sous forme de beaux cristaux blancs PF = 100,5-102,5 °C, a une toxicité exprimée en dose léthale 50 souris par voie orale, DL 50 souris P.O de 3 000 mg/kg (dénomination commune « idrocilamide ».

Le composé II : N-(hydroxy-2 éthyl) chloro-4 cinnamamide, $C_{11}H_{12}ClNO_2$, M = 225,68, cristallise sous forme d'aiguilles blanches, PF = 137-138,5 °C, DL 50 souris P.O 1 600 mg/kg.

Le composé III : N-méthyl, N-(hydroxy-2 éthyl) cinnamamide, $C_{12}H_{15}NO_2$, M = 205,25, cristallise sous forme de poudre blanche PF = 79-81 °C, DL 50 souris P.O 3 000 mg/kg.

Le composé IV : N-[(hydroxy-1 propyl)-2] cinnamamide, $C_{12}H_{15}NO_2$, M = 205,25, petites paillettes blanches PF = 144-146 °C, DL 50 souris P.O 3 200 mg/kg.

Le composé V : N-(hydroxy-2 éthyl) fluoro-4 cinnamamide, $C_{11}H_{12}FNO_2$, M = 209,22, PF = 100-102 °C, DL 50 souris P.O supérieure à 3 200 mg/kg.

Le composé VI : N-(hydroxy-2 éthyl) acétyl-4 cinnamamide, $C_{13}H_{15}NO_3$, M = 233,26, PF = 124-125 °C, DL 50 souris, P.O 3 200 mg/kg.

Les composés V et VI sont des produits chimiques nouveaux non décrits dans le brevet français 2.040.181.

Les dérivés pour lesquels -N $R_1R_2$ représente le groupe diméthyl-2,2 oxazolidinyl-3, peuvent se préparer en condensant dans un premier temps, l'éthanolamine sur l'acétone puis en faisant réagir un chlorure de cinnamoyle IV sur l'oxazolidine intermédiaire, qu'il n'est pas nécessaire d'isoler. La réaction s'effectue avantageusement avec un excès d'éthanolamine.

Le composé VII : (chloro-4 cinnamoyl)-3 diméthyl-2,2 oxazolidine, $C_{14}H_{16}ClNO_2$, M = 265,73, sous forme de cristaux blancs fond à 107,5-109,5 °C, DL 50 souris P.O supérieure à 3 200 mg/kg.

Le composé VIII : diméthyl-2,2 fluoro-4 cinnamoyl)-3 oxazolidine, $C_{14}H_{16}FNO_2$, M = 249,27, F = 106-108 °C, DL 50 souris P.O 2 000 mg/kg.

Le composé IX : cinnamoyl-3 diméthyl-2,2 oxazolidine, $C_{14}H_{17}NO_2$, M = 231,29, F = 107-109 °C, DL 50 souris P.O supérieure à 3 200 mg/kg.

Il a été trouvé et mis au point un modèle animal permettant de mettre en évidence l'action prolongée de la théophylline associée au N-(hydroxy-2 éthyl) cinnamamide.

Des rats mâles, de souche Wistar, pesant 220-240 g, à jeun de nourriture depuis 16 heures, reçoivent une administration orale de théophylline en solution dans l'eau.

La moitié des animaux reçoit, en même temps que la théophylline, l'idrocilamide en suspension dans la gomme arabique à 5 %, l'autre moitié recevant la gomme arabique seule. 15', 30', 1 h, 2 h, 3 h, 4 h, 6 h, 8 h, après administration de la théophylline, les animaux sont tués par section carotidienne. Le sang est recueilli dans des tubes héparinés et centrifugés, le plasma est décanté. La théophylline est dosée dans les échantillons plasmatiques après extraction par une méthode de chromatographie liquide.

Les moyennes des théophyllinémies obtenues, ainsi que les paramètres pharmacocinétiques calculés, sont donnés dans le tableau 1 ci-dessous.

Tableau 1

| Concentration en mg/l de théophylline à | Animaux ayant reçu la théophylline seule Moyenne (écart-type) | Animaux ayant reçu la théophylline + idrocilamide Moyenne (écart-type) | |
|---|---|---|---|
| 15' | 17,54 (5,77) | 14,94 (3,19) | NS |
| 30' | 20,26 (4,88) | 16,68 (2,29) | NS |
| 1 h | 18,86 (3,09) | 15,79 (1,39) | NS |
| 2 h | 16,69 (1,95) | 17,26 (3,46) | NS |
| 3 h | 13,93 (2,05) | 17,57 (2,21) | S |
| 4 h | 11,96 (2,43) | 17,74 (1,53) | S |
| 6 h | 8,04 (2,17) | 14,10 (2,81) | S |
| 8 h | 4,32 (1,91) | 11,51 (3,26) | S |
| Surface → 8 h mg/l-1 h | 95,10 | 123 | |
| Demi-vie plasmatique en h | 3,47 | 6,42 | |
| Surface → ∞ mg/l-1 h | 116,70 | 229,60 | |

NS = non significatif, S = significatif.

Ce tableau appelle les constatations suivantes :

1) La concentration maximale de théophylline n'est pas augmentée : 20,26 pour la théophylline seule, 17,74 pour l'association avec « l'idrocilamide » ;

2) Les concentrations de théophylline obtenues avec l'association sont significativement supérieures à celles obtenues avec la théophylline seule dès 3 heures après l'administration et le reste jusqu'à la fin de l'essai. A 8 heures la concentration est 2,7 fois plus élevée ;

3) la demi-vie calculée à partir du maximum est augmentée de 85 % par l'administration simultanée d'« idrocilamide » ;

4) La surface sous la courbe des concentrations en fonction du temps est augmentée de 29,4 % jusqu'à huit heures et de 166 % si extrapolée à l'infini.

Sur la figure I du dessin annexé sont représentées les courbes des concentrations de théophylline en fonction du temps après une administration de théophylline seule ou avec l'« idrocilamide », et l'on peut découvrir la stabilité de la théophyllinémie après association avec l'idrocilamide et l'augmentation considérable de la biodisponibilité. Le temps en heures est porté sur l'axe des abscisses, t(H) et la concentration en mg/l de théophylline Th mg/l sur l'axe des ordonnées. La courbe I correspond à la théophylline seule et la courbe II à l'association théophylline + « idrocilamide ».

Ce modèle a permis de découvrir qu'un certain nombre d'analogues structuraux du N-(hydroxy-2 éthyl) cinnamamide possède chez l'animal, les mêmes propriétés d'accroissement considérable des surfaces sous la courbe de la théophyllinémie en fonction du temps, lorsqu'ils sont administrés en associés à la théophylline. Divers composés concernés par l'invention ont donné, dans le test décrit précédemment, les résultats consignés dans le tableau 2, ci-dessous.

Tableau 2

| | Concentration maximum mg/l | Concentration à 8 h mg/l | Demi-vie plasmatique heure | Surface → ∞ mg/l-1 h |
|---|---|---|---|---|
| Composé I | 17,74 | 11,51 | 6,42 | 229,60 |
| Composé II | 16,80 | 12,75 | 10 | 306,70 |
| Composé III | 20,50 | 7,58 | 4,50 | 160 |
| Composé IV | 19,70 | 11,86 | 9,20 | 286 |
| Composé V | 22,30 | 15,46 | 16,10 | 501,70 |
| Composé VI | 18 | 10,27 | 10 | 269,40 |
| Composé VII | 21,30 | 14,81 | 11,40 | 385,60 |
| Composé VIII | 21,17 | 11,93 | 8,5 | 294 |
| Composé IX | 22,5 | 13,36 | 7,3 | 288,7 |

L'association de la théophylline et d'un composé cinnamamide substitué selon l'invention constitue un médicament utilisable dans les applications de la théophylline et dérivés dans le traitement de l'asthme.

L'association de la théophylline avec un composé cinnamamide substitué par le groupe diméthyloxazolidinyl s'avère très intéressante, en particulier celle avec le (chloro-4 cinnamoyl)-3 diméthyl-2,2 oxazolidine et cinnamoyl-3 diméthyl-2,2 oxazolidine.

Les proportions du dérivé cinnamamide substitué dans l'association sont adaptées, en particulier à l'âge et au poids des malades. Ce dérivé exerce une action au niveau de la métabolisation de la théophylline, même à faible dose. Il peut être associé à la théophylline dans un rapport pouvant varier de 0,1 à 4 suivant le sujet et la forme galénique.

La théophylline peut être introduite dans l'association sous forme libre ou combinée par exemple avec l'éthylène diamine (euphylline®, aminophylline®). On peut envisager l'emploi en addition de théophylline et aminophylline®.

La dose unitaire en association principe actif théophylline ou dérivé + cinnamamide substitué peut varier entre 50 et 1 200 mg. On peut proposer un traitement en deux prises par jour espacées de douze heures.

Il est donné ci-après, à titre illustratif, quelques formulations associant la théophylline ou l'aminophylline® à un composé cinnamamide substitué selon l'invention, utilisables en thérapeutique. Les proportions du dérivé cinnamamide dans l'association sont variables afin de tenir compte, en particulier, de l'âge et du poids des malades.

n° 1 — Comprimés

| | |
|---|---|
| Théophylline | 200 mg |
| Cinnamamide substitué | 200 mg |
| Lactose | 34 mg |
| Amidon | 33 mg |
| Gélatine | 10 mg |
| Acide alginique | 20 mg |
| Stéarate de magnésium | 3 mg |
| | 500 mg |

n° 2 — « Comprimés sécables en bâtonnets »

| | |
|---|---|
| Théophylline | 200 mg |
| Cinnamamide substitué | 400 mg |
| Lactose | 50 mg |
| Amidon | 50 mg |
| Gélatine | 15 mg |
| Acide alginique | 30 mg |
| Stéarate de magnésium | 5 mg |
| | 750 mg |

n° 3 — Comprimés dragéifiés

| | | |
|---|---|---|
| Théophylline | 200 mg | 100 mg |
| Cinnamamide substitué | 100 mg | 100 mg |
| Lactose | 37 mg | 40 mg |
| Amidon | 36 mg | 40 mg |
| Gélatine | 8 mg | 6 mg |
| Acide alginique | 16 mg | 12 mg |
| Stéarate de magnésium | 3 mg | 2 mg |
| | 400 mg | 300 mg |
| Enrobage de dragéification qs | 600 mg | 500 mg |

Enrobage de dragéification : sucre, gomme arabique, gélatine, talc, cire blanche.

n° 4 — Comprimés gastrorésistants

Préparation des comprimés selon la formule n° 3. Avant dragéification les comprimés sont rendus gastrorésistants par application de phtalate d'hydroxypropylméthylcellulose en solution dans l'acétone-isopropanol.

n° 5 — Suppositoires

Aminophylline® 300 mg
Cinnamamide substitué 100 mg
Glycérides semi-synthétiques qsq 1 suppositoire d'environ 3 g.

n° 6 — Solution injectable

| | | |
|---|---|---|
| Théophylline | 100 mg | 50 mg |
| Cinnamamide substitué | 15 mg | 15 mg |
| Anisate de sodium | 300 mg | 150 mg |
| Ethanol à 96° | 0,25 ml | 0,25 ml |
| eau ppi qsq | 5 ml | 5 ml |

On a conduit une série d'essais cliniques comparatifs. A cinq malades asthmatiques, adultes de sexe masculin, on a administré par voie orale deux fois par jour, à 8 heures et à 20 heures, une prise simultanée de 200 mg de théophylline et de 100 mg d'idrocilamide. Le 4e jour, après l'établissement de l'état d'équilibre, on a dressé à différents temps le taux sanguin de théophylline chez les malades ainsi traités. Le dosage a été réalisé par chromatographie liquide haute pression (appareil de Waters — adsorbant Lichrosorb RP 18 de Merck — Solvant phosphate monopotassique, formamide, méthanol. Détection par spectrophotométrie (UV). Les moyennes des valeurs obtenues figurent dans le tableau 3 ci-dessous

Tableau 3

| Temps | Concentration sanguine en théophylline |
|---|---|
| avant la prise du matin le 4e jour | 9,5 mg/l |
| 2 heures après la prise du matin | 11,5 mg/l |
| 4 heures après | 12 mg/l |
| 8 heures après | 11,5 mg/l |
| 12 heures après | 10 mg/l |

On peut faire les constatations suivantes :

1) Le matin, avant toute prise du médicament le taux sanguin de théophylline est de 9,5 mg/l, c'est-à-dire dans une zone de concentration active. Comme il s'agit de la concentration la plus basse, puisque la plus éloignée de la prise du médicament, cela signifie que pendant toute la nuit, la concentration sanguine a été suffisante pour assurer l'activité thérapeutique.

2) Pendant ce 4e jour, après la prise du matin, le taux sanguin subit une augmentation modérée, restant globalement dans une zone comprise entre 10 et 15 mg/l. Le taux sanguin est donc stable.

3) 12 heures après la prise du matin, et avant la prise du soir, le taux sanguin est encore de 10 mg/l, taux suffisant pour assurer l'action thérapeutique.

On peut donc conclure qu'avec cette association théophylline + « idrocilamide » on obtient un taux stable et prolongé de théophylline permettant une efficacité thérapeutique de 24 heures sur 24.

Ces résultats sont à comparer à ceux obtenus, dans les mêmes conditions, avec l'administration de 200 mg de théophylline seule ; ils sont résumés dans le tableau 4 ci-dessous.

Tableau 4

| Temps | Concentration sanguine en théophylline |
|---|---|
| avant la prise du matin le 4e jour | 2 mg/l |
| 2 heures après la prise du matin | 7,5 mg/l |
| 4 heures après | 10,5 mg/l |
| 8 heures après | 5,5 mg/l |
| 12 heures après | 1,5 mg/l |

Le graphique représenté sur la figure 2 du dessin annexé récapitule et visualise les résultats obtenus le 4e jour quand on administre à l'homme, deux fois par jour à 12 heures d'intervalle, la même dose de 200 mg de théophylline seule ou associée à 100 mg d'« idrocilamide ». Le temps en heures (t) après la prise du matin est porté en abscisses, le temps 0 correspondant à la prise du médicament au 4e jour. Les concentrations sanguines de théophylline en mg/l (th mg/l) sont portées en ordonnées. La courbe I correspond aux résultats après une prise de 200 mg de théophylline, la courbe II correspond aux résultats après une prise du médicament constitué par 200 mg de théophylline associée à 100 mg d'« idrocilamide ».

Outre qu'un taux sanguin prolongé soit obtenu grâce à l'association de l'« idrocilamide » à la théophylline, on constate que l'aire sous la courbe II est bien supérieure pour l'association, alors qu'une même dose de 200 mg de théophylline a été administrée dans les deux cas.

Puisque l'absorption de la théophylline par la voie orale est très bonne, cette constatation indique que le mécanisme d'action est lié à une interférence de l'« idrocilamide » sur le métabolisme de la théophylline dont la demi-vie se trouve ainsi prolongée. Chez les adultes à fonction rénale normale, on sait que la posologie de la théophylline varie d'un sujet à l'autre, malgré la bonne absorption du médicament. C'est que cette variation provient d'une différence dans la capacité individuelle de métabolisation. L'« idrocilamide » agissant au niveau de cette métabolisation, l'association permettra une meilleure uniformisation des posologies.

Par la suite quinze autres malades asthmatiques ont été traités avec l'association théophylline-idrocilamide. Il n'y a que peu de problème de tolérance et les résultats thérapeutiques ont été bons. Dans quelques cas, des résultats particulièrement favorables ont été obtenus, avec sevrage des corticoïdes.

Il est donné, à titre non limitatif, des exemples de préparation des nouveaux produits chimiques.

Exemple 1 : (Chloro-4 cinnamoyl)-3 diméthyl-2,2 oxazolidine

On ajoute goutte à goutte 13,4 g (0,22 mole) d'éthanolamine à 30 $cm^3$ d'acétone. Le mélange s'échauffe. Lorsqu'il est revenu à température ambiante, on ajoute, en maintenant la température entre 15 et 20 °C, une solution de 18,3 g (0,09 mole) de chlorure de chloro-4 cinnamoyle dans 30 $cm^3$ d'acétone puis agite 2 heures à température ambiante. Le milieu réactionnel est dilué à l'eau. La (chloro-4 cinnamoyl)-3 diméthyl-2,2 oxazolidine se sépare sous forme de cristaux blancs. Elle est filtrée, lavée à l'eau, séchée et recristallisée dans l'heptane. F = 107,5-109,5 °C.

Analyse centésimale $C_{14}H_{16}ClNO_2$ M = 265,73
Calculé : C % 63,29 H % 6,07 Cl % 13,35 N % 5,27
Trouvé : C % 63,16 H % 6,20 Cl % 13,40 N % 5,28
I.R. (KBr) $\nu$ (C = O) = 1 660 $cm^{-1}$
R.M.N. ($CDCl_3$) $\delta$ = 1,7 ppm (singulet, 4H) ;
3,6-4,3 (multiplet, 4H) ;
6,6 (doublet, J = 14 Hz, 1H) ;
7,2-7,6 (massif complexe, 4H) ;
7,6 (doublet, J = 14 Hz, 1H).

Exemple 2 : N-(hydroxy-2 éthyl) fluoro-4 cinnamamide

Une solution de 374 g (2,03 moles) de chlorure de fluoro-4 cinnamoyle dans 2 l de dioxanne est ajoutée goutte à goutte à 20 °C, à une solution de 297 g (4,87 moles) d'éthanolamine dans 2 l de dioxanne. Le milieu réactionnel est agité 3 heures à température ambiante, abandonné une nuit et

concentré sous pression réduite. Le résidu est repris par une solution de bicarbonate de sodium. Le précipité de N-(hydroxy-2 éthyl) fluoro-4 cinnamamide est filtré, lavé à l'eau et séché. Rdt 351 g (soit 83 % de la théorie). Le produit est purifié par recristallisation dans l'acétate d'éthyle. F = 100-102 °C.

Analyse centésimale $C_{11}H_{12}FNO_2$ M = 209,22
Calculé : C % 63,14 H % 5,78 F % 9,08 N % 6,70
Trouvé : C % 63,09 H % 5,68 F % 9,32 N % 6,72
I.R. (KBr) : $\nu$ (C = O) = 1 660 $cm^{-1}$

Exemple 3 : N-(hydroxy-2 éthyl- acétyl-4 cinnamamide

Un mélange de 9,5 g (0,05 mole) d'acide acétyl-4 cinnamique [obtenu selon G.H. Cleland J. Org. Chem. *34*, 744 (1969)] de 30 g de chlorure de thionyle et de 40 $cm^3$ de benzène est porté 20 minutes à reflux. La solution est concentrée à sec sous pression réduite. Le solide résiduel est dissous dans 100 $cm^3$ de dioxanne et la solution obtenue est ajoutée goutte à goutte, à une température de 20 °C à une solution de 9,3 g (0,15 mole) d'éthanolamine dans 50 $cm^3$ de dioxanne. Le mélange est agité 1 heure à température ambiante, versé dans un mélange de 500 g de glace et de 20 $cm^3$ d'acide chlorhydrique 10N puis extrait au chloroforme. La phase organique est lavée à l'eau et séchée sur sulfate de sodium. Par évaporation du solvant sous pression réduite on obtient le N-(hydroxy-2 éthyl) acétyl-4 cinnamamide qui est purifié par recristallisation dans l'acétate d'éthyle. F = 124-125 °C Rdt : 4,2 g (36 % de la théorie).

Analyse centésimale $C_{13}H_{15}NO_3$ M = 233,26
Calculé : C % 66,93 H % 6,48 N % 6,01
Trouvé : C % 66,80 H % 6,35 N % 6,12
I.R. (KBr) : $\nu$ (C = O) = 1 680 et 1 660 $cm^{-1}$

Exemple 4 : Diméthyl-2,2 (fluoro-4 cinnamoyl)-3 oxazolidine

En opérant comme dans l'exemple 1 à partir de chlorure de fluoro-4 cinnamoyle, on obtient la (fluoro-4 cinnamoyl)-3 diméthyl-2,2 oxazolidine. Rdt : 77 %. F = 106-108 °C (hexane-acétate d'éthyle).

Analyse centésimale $C_{14}H_{16}FNO_2$ M = 249,27.
Calculé : C % 67,45 H % 6,47 F % 7,62 N % 5,62
Trouvé : C % 67,43 H % 6,65 F % 7,71 N % 5,73

Exemple 5 : Cinnamoyl-3 diméthyl-2,2 oxazolidine Composé VIII

En opérant comme dans l'exemple 1 à partir du chlorure de cinnamoyle, on obtient la cinnamoyl-3 diméthyl-2,2 oxazolidine. Rdt : 74 %. F = 107-109 °C (hexane-acétate d'éthyle).

Analyse centésimale $C_{14}H_{17}NO_2$ M = 231,29
Calculé : C % 72,69 H % 7,41 N % 6,06
Trouvé : C % 72,62 H % 7,42 N % 6,05

**Revendications** (pour les Etats contractants : BE, CH, DE, FR, GB, IT, LI, LU, NL, SE)

1. Médicament à base de théophylline, à action prolongée, caractérisé par l'association de la théophylline (diméthyl-1,3 xanthine), euphylline® ou aminophylline®, à un composé du type cinnamamide substitué répondant à la formule générale

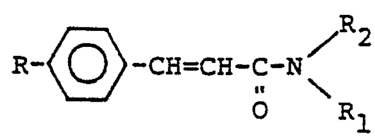

(I)

dans laquelle R est l'hydrogène, un halogène ou un radical acétyle ; $R_1$ est l'hydrogène ou le radical méthyle : $R_2$ représente un groupe de formule II

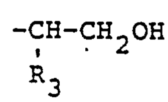

dans laquelle $R_3$ est l'hydrogène ou le radical méthyle ; $R_1$ et $R_2$ peuvent également former avec l'atome d'azote adjacent, le groupe diméthyl-2,2 oxazolidinyl-3.

2. Médicament à base de théophylline, selon la revendication 1, caractérisé par l'association de la théophylline, euphylline® ou aminophylline® au (chloro-4 cinnamoyl-3 diméthyl-2,2 oxazolidine).

3. Médicament à base de théophylline, selon la revendication 1, caractérisé par l'association de la théophylline, de l'euphylline® ou de l'aminophylline® au cinnamoyl-3 diméthyl-2,2 oxazolidine.

4. Médicament selon la revendication 1, caractérisé en ce que la dose unitaire en association principe actif théophylline, euphylline® ou aminophylline® + « idrocilamide » est comprise entre 50 et 1 200 mg.

5. Médicament selon la revendication 1, caractérisé en ce que la xanthine introduite dans l'association est la théophylline sous forme libre ou combinée avec l'éthylènediamine dite aminophylline® seule ou en mélange.

6. Médicament selon la revendication 1, caractérisé en ce qu'il est présenté sous forme de comprimés, ampoules injectables ou suppositoires.

7. Médicament selon la revendication 1, utilisable dans le traitement de l'asthme.

8. Cinnamamide substitué caractérisé par la formule

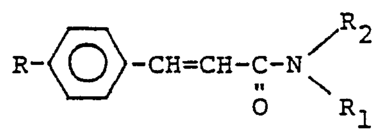

dans laquelle R est l'hydrogène ou un halogène, $R_1$ et $R_2$ forment avec l'atome d'azote adjacent le groupe diméthyl-2,2 oxazolidinyl-3.

9. N-(hydroxy-2 éthyl) acétyl-4 cinnamamide.

**Revendications** (pour l'Etat contractant AT)

1. Procédé de préparation d'une composition à base de théophylline, caractérisé en ce qu'on procède à l'association de la théophylline (diméthyl-1,3 xanthine), euphylline® ou aminophylline®, à un composé du type cinnamamide substitué répondant à la formule générale

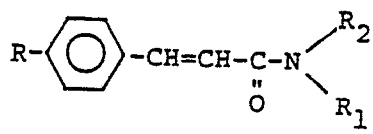

dans laquelle R est l'hydrogène, un halogène ou un radical éthyle ; $R_1$ est l'hydrogène ou le radical méthyle ; $R_2$ représente un groupe de formule II

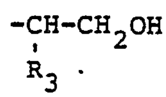

dans laquelle $R_3$ est l'hydrogène ou le radical méthyle ; $R_1$ et $R_2$ peuvent également former avec l'atome d'azote adjacent, le groupe diméthyl-2,2-oxazolidinyl-3, dans un rapport de 0,1 à 4.

2. Procédé de préparation des cinnamamides substitués représentés par la formule

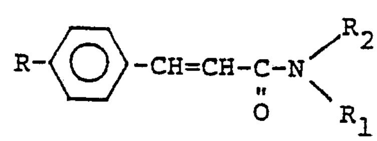

dans laquelle R est l'hydrogène ou un halogène et $R_1$ et $R_2$ forment avec l'atome d'azote adjacent le groupe diméthyl-2,2 oxazolidinyl-3, caractérisé par la condensation de l'éthanolamine sur l'acétone, puis en la réaction d'un chlorure de cinnamoyle.

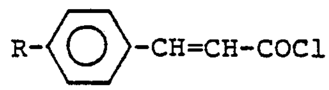

sur l'oxazolidone intermédiaire.

3. Procédé de préparation des cinnamamides substitués selon la revendication 2, caractérisé en ce que la réaction est conduite en présence d'un excès d'éthanolamine.

4. Procédé de préparation du (chloro-4 cinnamoyl)-3 diméthyl-2,2 oxazolidine, caractérisé en ce que l'on ajoute l'éthanolamine dans l'acétone, puis à la température ambiante on introduit une solution de chlorure de chloro-4 cinnamoyle dans l'acétone.

5. Procédé de préparation du diméthyl-2,2 (fluoro-4 cinnamoyl)-3 oxazolidine caractérisé en ce que l'on fait réagir le chlorure de fluoro-4 cinnamoyle sur l'oxazolidone intermédiaire obtenue par condensation de l'éthanolamine sur l'acétone.

6. Procédé de préparation du cinnamoyl-3 diméthyl-2,2 oxazolidine caractérisé en ce que l'on fait réagir le chlorure de cinnamoyle sur l'oxazolidone intermédiaire obtenue par condensation de l'éthanolamine sur l'acétone.

**Claims** (for the Contracting States : BE, CH, DE, FR, GB, IT, LI, LU, NL, SE)

1. Sustained release theophylline based drug, characterized by the combination of theophylline (1,3-dimethylxanthine), euphylline® or aminophylline® with a compound of the substituted cinnamic acid amide type of the general formula

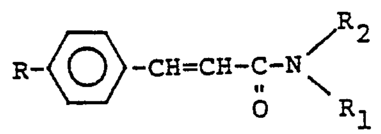

(I)

wherein R is hydrogen, halogen or an acetyl radical, $R_1$ is hydrogen or the methyl radical, $R_2$ is a group of the formula II

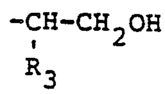

(II)

wherein $R_3$ is hydrogen or the methyl radical, and $R_1$ and $R_2$ can also form the 2,2-dimethyl-3-oxazolidinyl group together with the adjacent hydrogen atom.

2. Theophylline based drug according to claim 1, characterized by the combination of the theophylline, euphylline® or aminophylline® with 4-chloro-3-cinnamoyl-2,2-dimethyl oxazolidine.

3. Theophylline based drug according to claim 1, characterized by the combination of the theophylline, euphylline® or aminophylline® with 3-cinnamoyl-2,2-dimethyl oxazolidine.

4. Drug according to claim 1, characterized in that the unit dose of the combination of the active principles theophylline, euphylline® or aminophylline® + « Idrocilamid » is between 50 and 1 200 mg.

5. Drug according to claim 1, characterized in that the xanthene introduced into the combination is theophylline in the free form or combined with ethylene diamine called aminophylline®, solely or in admixture.

6. Drug according to claim 1, characterized in that it has the form of tabletts, injectable ampoules or suppositories.

7. Drug according to claim 1, to be used for the treatment of asthma.

8. Substituted cinnamic acid amide characterized by the formula

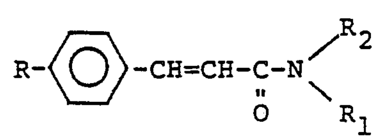

wherein R is hydrogen or halogen and $R_1$ and $R_2$ form the 2,2-dimethyl-3-oxazolidinyl group together with the adjacent nitrogen atom.

9. N-(2-hydroxyethyl)-4-acetyl cinnamic acid amide.

**Claims** (for the Contracting State AT)

1. Process for the preparation of a theophylline based composition, characterized in that theophylline (1,3-dimethyl xanthine), euphylline® or aminophylline® is combined with a compound of the substituted cinnamic acid amide type of the general formula

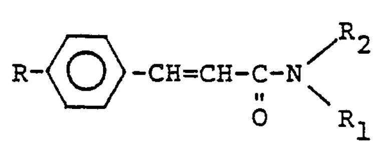

wherein R is hydrogen, halogen or an ethyl radical, $R_1$ is hydrogen or the methyl radical, $R_2$ is a group of the formula II

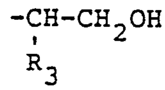

wherein $R_3$ is hydrogen or the methyl radical and $R_1$ and $R_2$ can also form the 2,2-dimethyl-3-oxazolidinyl group together with the adjacent nitrogen atom, in a ratio of 0.1 to 4.

2. Process for preparation of substituted cinnamic acid amides of the formula

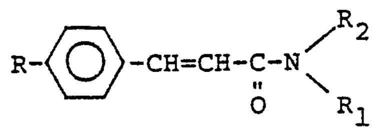

wherein R is hydrogen or halogen and $R_1$ and $R_2$ form the 2,2-dimethyl-3-oxazolidinyl group together with the adjacent nitrogen atom, characterized by the condensation of ethanol amine with acetone and subsequent reaction of a cinnamoyl chloride of the formula

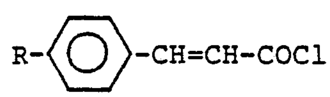

with the intermediate oxazolidone.

3. Process for preparation of substituted cinnamic acid amides according to claim 2, characterized in that the reaction is carried out in presence of an excess of ethanol amine.

4. Process for preparation of 3-(4-chlorocinnamoyl)-2,2-dimethyl oxazolidine, characterized in that ethanol amine is added to acetone and then a solution of 4-chlorocinnamoyl choride is introduced at ambient temperature into the acetone.

5. Process for preparation of 2,2-dimethyl-3-(4-fluoro cinnamoyl)-oxazolidine, characterized in that 4-fluoro cinnamoyl chloride is reacted with the intermediate oxazolidone obtained by condensation of ethanolamine with acetone.

6. Process for preparation of 3-cinnamoyl-2,2-dimethyl oxazolidine, characterized in that cinnamoyl chloride is reacted with the intermediate oxazolidone obtained by condensation of ethanole amine with acetone.

**Patentansprüche** (für die Vertragsstaaten : BE, CH, DE, FR, GB, IT, LI, LU, NL, SE)

1. Arzneimittel auf Theophyllinbasis mit verlängerter Wirkung, gekennzeichnet durch die Kombination des Theophyllins (1,3-Dimethylxanthin), Euphyllins® oder Aminophyllins® mit einer Verbindung vom substituierten Zimtsäureamidtyp entsprechend der allgemeinen Formel

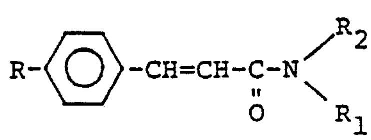

(I)

worin R ein Wasserstoffatom, ein Halogenatom oder ein Acetylrest ist, $R_1$ ein Wasserstoffatom oder der Methylrest ist, $R_2$ eine Gruppe der Formel II

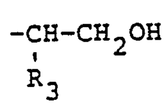

(II)

bedeutet, worin $R_3$ ein Wasserstoffatom oder der Methylrest ist, und $R_1$ und $R_2$ auch zusammen mit dem benachbarten Stickstoffatom die 2,2-Dimethyl-3-oxazolidinylgruppe bilden können.

2. Arzneimittel auf Theophyllinbasis nach Anspruch 1, gekennzeichnet durch die Kombination des Theophyllins, Euphyllins® oder Aminophyllins® mit 4-Chlor-3-cinnamoyl-2,2-dimethyloxazolidin.

3. Arzneimittel auf Theophyllinbasis nach Anspruch 1, gekennzeichnet durch die Kombination des Theophyllins, des Euphyllins® oder des Aminophyllins® mit 3-Cinnamoyl-2,2-dimethyloxazolidin.

4. Arzneimittel nach Anspruch 1, dadurch gekennzeichnet, daß die Einheitsdosis der Wirkstoffkom-

bination von Theophyllin, Euphyllin® oder Aminophyllin® plus « Idrocilamid » zwischen 50 und 1 200 mg liegt.

5. Arzneimittel nach Anspruch 1, dadurch gekennzeichnet, daß das in die Kombination eingeführte Xanthin Theophyllin in freier Form oder mit Äthylendiamin vereinigt, genannt Aminophyllin®, allein oder im Gemisch ist.

6. Arzneimittel nach Anspruch 1, dadurch gekennzeichnet, daß es in der Form von Tabletten, injizierbaren Ampullen oder Suppositorien vorliegt.

7. Arzneimittel nach Anspruch 1, das bei der Behandlung von Asthma verwendbar ist.

8. Substituiertes Zimtsäureamid der Formel

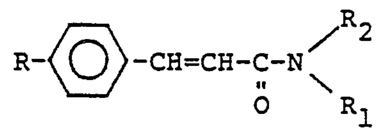

worin R ein Wasserstoffatom oder ein Halogenatom bedeutet und $R_1$ und $R_2$ mit dem benachbarten Stickstoffatom die 2,2-Dimethyl-3-oxazolidinylgruppe bilden.

9. N-(2-Hydroxyäthyl)-4-acetylzimtsäureamid.

**Patentansprüche** (für den Vertragsstaat AT)

1. Verfahren zur Herstellung einer Zusammensetzung auf Theophyllinbasis, dadurch gekennzeichnet, daß man Theophyllin (1,3-Dimethylxanthin), Euphyllin® oder Aminophyllin® in einem Mengenverhältnis von 0,1 bis 4 mit einer Verbindung vom substituierten Zimtsäureamidtyp der allgemeinen Formel

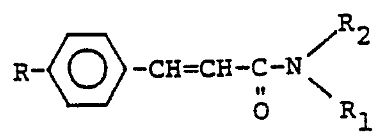

vereinigt, worin R ein Wasserstoffatom, ein Halogenatom oder eine Äthylgruppe ist, $R_1$ ein Wasserstoffatom oder die Methylgruppe ist, $R_2$ eine Gruppe der Formel II

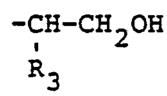

bedeutet, worin $R_3$ ein Wasserstoffatom oder die Methylgruppe bedeutet, und $R_1$ und $R_2$ auch mit dem benachbarten Stickstoffatom die 2,2-Dimethyl-3-oxazolidinylgruppe bilden können.

2. Verfahren zur Herstellung substituierter Zimtsäureamide der Formel

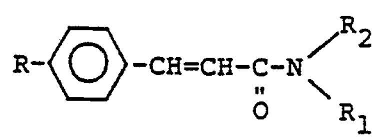

worin R ein Wasserstoffatom oder ein Halogenatom ist und $R_1$ und $R_2$ zusammen mit dem benachbarten Stickstoffatom die 2,2-Dimethyl-3-oxazolidinylgruppe bilden, gekennzeichnet durch die Kondensation von Äthanolamin mit Aceton und anschließende Umsetzung eines Zimtsäurechlorids der Formel

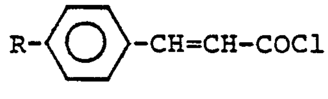

mit dem Oxazolidon-Zwischenprodukt.

3. Verfahren zur Herstellung substituierter Zimtsäureamide nach Anspruch 2, dadurch gekennzeichnet, daß die Umsetzung in Gegenwart eines Äthanolaminüberschusses durchgeführt wird.

4. Verfahren zur Herstellung von 3-(4-Chlorcinnamoyl)-2,2-dimethyloxazolidin, dadurch gekennzeichnet, daß man Äthanolamin in Aceton gibt und dann bei Umgebungstemperatur eine Lösung von 4-Chlorzimtsäurechlorid in das Aceton einführt.

5. Verfahren zur Herstellung von 2,2-Dimethyl-3-(4-fluorcinnamoyl)-oxazolidin, dadurch gekennzeichnet, daß man 4-fluorzimtsäurechlorid mit dem Oxazolidon-Zwischenprodukt, das man durch Kondenation von Äthanolamin mit Aceton erhalten hat, reagieren läßt.

6. Verfahren zur Herstelllung von 3-Cinnamoyl-2,2-dimethyl-oxazolidin, dadurch gekennzeichnet, daß man Zimtsäurechlorid mit dem Oxazolidon-Zwischenprodukt, das man durch Kondensation von Äthanolamin mit Aceton erhalten hat, reagieren läßt.

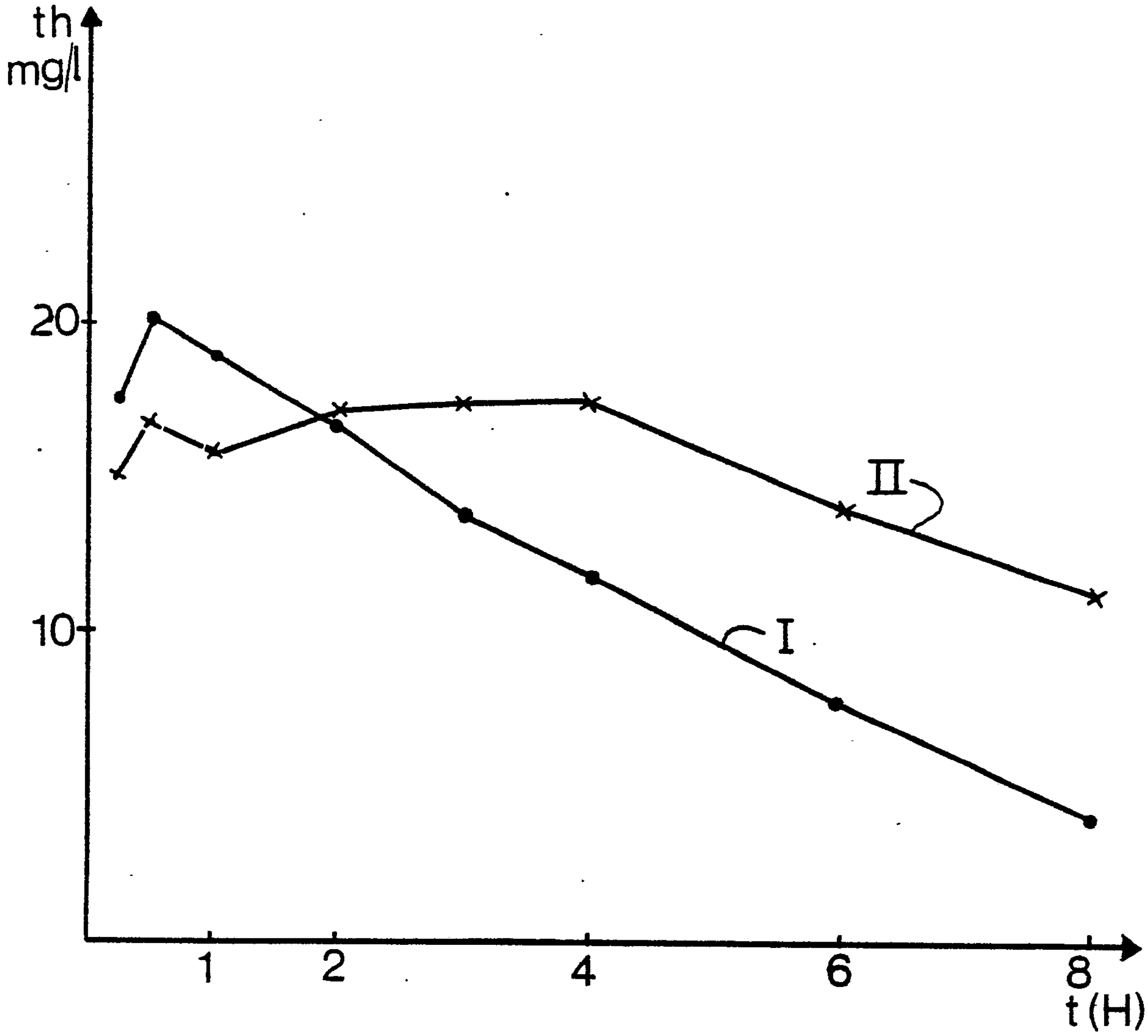


FIG.1

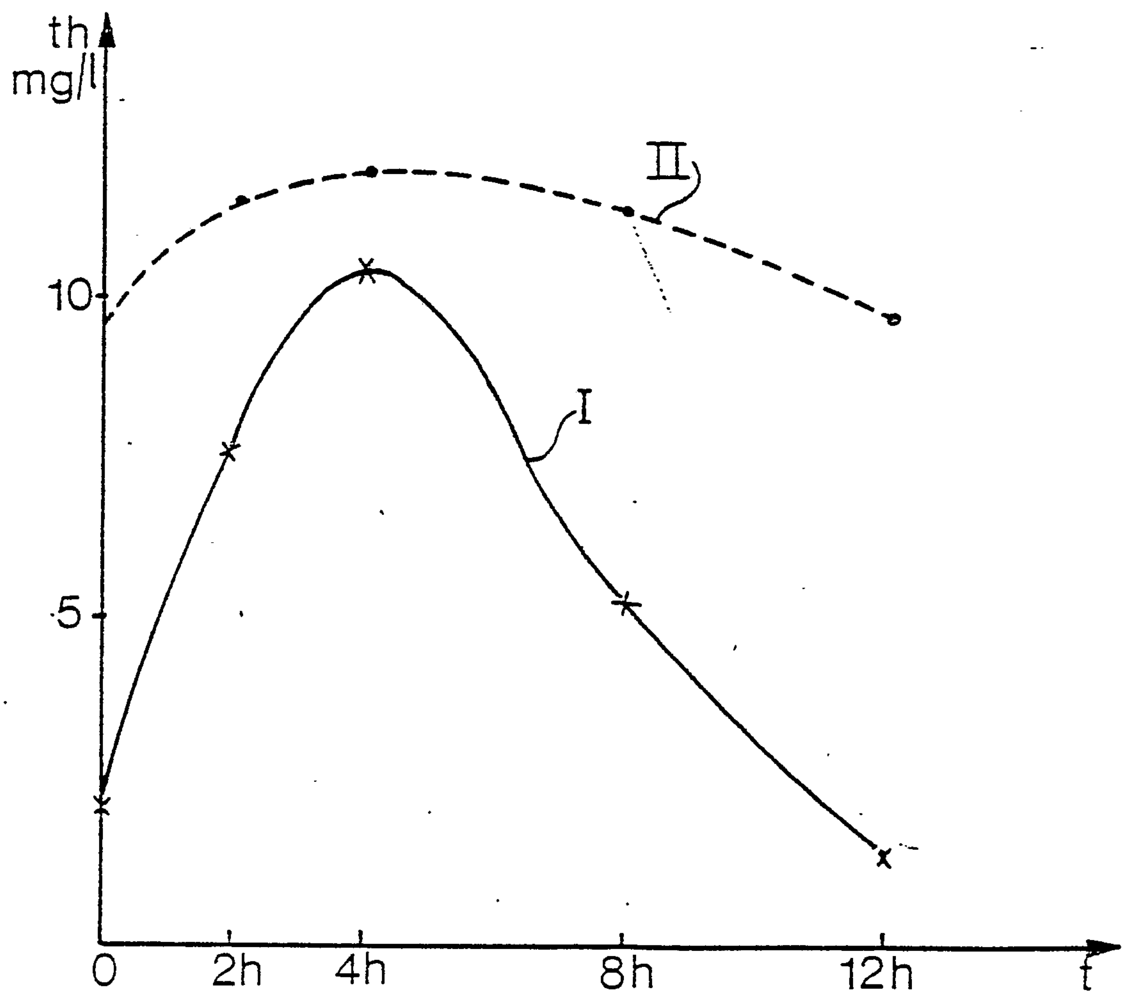


FIG.2